# EUROPEAN PATENT SPECIFICATION

(11) **EP 1 046 719 B1**
(45) Date of publication and mention of the grant of the patent: **26.05.2004**
(21) Application number: 99201250.0
(22) Date of filing: 20.04.1999
(51) Int. Cl.: C13K 13/00, C12P 19/02, C12P 19/16

(54) **D-galactose composition and process for its manufacture**
D-galactosezusammensetzung und Verfahren zu ihrer Herstellung
Composition de D-galactose et procédé pour sa fabrication

(43) Date of publication of application: 25.10.2000
(73) Proprietor: Cargill B.V., 1005 AB Amsterdam (NL)
(72) Inventor: Mollee, Peter Wilma, 4845 CT Wagenberg (NL); Kempener, Saskia, 4611 WH Bergen op Zoom (NL)
(74) Representative: van Westenbrugge, Andries

(56) References cited:
- EP-A- 0 081 262
- EP-A- 0 168 127
- EP-A- 0 499 165
- FR-A- 1 555 370
- FR-A- 2 302 694
- US-A- 4 156 076

## Description

### FIELD OF INVENTION

The invention relates to procedures for the manufacturing of D-galactose and D-galactose containing compositions. More specifically, the invention provides a method for isolating D-galactose from legume material, which method comprises subjecting a legume composition containing non-homologous soluble polysaccharide, commonly referred to as oligosaccharide, to one or more treatments, resulting in a preparation comprising at least 30% oligosaccharides, and subsequent hydrolysis of the oligosaccharides in this preparation into mainly monosaccharides.

### BACKGROUND OF INVENTION

D-galactose is widely used as a raw material in industry. For example, many sweeteners, such as polyol sugars use D-galactose as a raw material for manufacture thereof. Examples are arabinose, pentitol, arabinitol, globotriose, arbitol, galactitol, xylitol and tagatose. Also D-galactose can be used in the beverage industry, e.g. in sport drinks, as replacement of phenols in resins, in the manufacturing of contrast agents, or as sweetener in foods, e.g. to prevent tooth decay.

Several methods for the manufacturing of D-galactose are known. One of the most conventional types of D-galactose manufacturing currently applied is the hydrolysis of milk sugar, lactose. Milk sugar can be isolated from for example milk whey. Over the last years, numerous patent applications regarding the optimization of D-galactose isolation from milk sugar have been disclosed. Examples of processes for the production of D-galactose from milk are mentioned below.US4156076 discloses a process for the conversion of lactose into monosaccharides and derivatives thereof, comprising oxidatively hydrolyzing a lactose solution to form galactose and gluconic acid, and separating these two constituents.

US 3981773 discloses a process for the preparation of D-galactose and beverages based on D-galactose from solutions containing lactose. The process comprises (1) the cultivation of a mutant, non-pathogenic prototrophic yeast or bacteria processing beta-galactosidase activity and a gal character on a media containing lactose and (2) the recovery of D-galactose.

US 4067748 discloses the hydrolysis of lactose from whey to glucose and D-galactose. Lactose is contacted in water with a solid, insoluble, strongly acidic ion exchange resin based on certain cross-linked polystyrenes or certain carbohydrates.
FR 2530661 discloses the hydrolysis of lactose contained in protein concentrates. The products are obtained by enzymatic hydrolysis, using lactases or beta-galactosidases, of the lactose in lactoprotein concentrates generated via ultrafiltration of whey.
EP168127 discloses a process and apparatus for continuous hydrolyzation of lactose into glucose and galactose employing a flow-through reactor upon which a lactase system has been immobilized. Introducing a continuous stream of a lactose solution, either from lactose powder crystallized from a whey stream or directly from one or more ultrafiltration devices, which remove the whey protein in a concentrated form from a whey stream and give a proper lactose solution.

EP499165 discloses a process for the production of a sugary syrup, containing galactose, fructose and glucose. The sugary syrup is prepared from whey ultrafiltrate, by purification, desalinization, concentration, hydrolysis of lactose, isomerisation of glucose into fructose and purification/concentration of the product.
In this process much effort has been put into the generation of a blend of monosaccharides comprising D-galactose, indicating that monosaccharide blends comprising D-galactose are highly desired. The monosaccharide blend presented in EP499165 can also be easily manufactured with the new process according to the present invention.

A disadvantage of using milk products as a raw material source for the production of D-galactose is partially of quantitative nature. Milk supply is limited and is not likely to increase over the coming years and is insufficient to supply industry with the current and future needs of D-galactose.

Additionally, the processing of D-galactose preparations from milk products needs to be extensive in order to prevent the preparation from being contaminated with intrinsic milk substances. Contamination could for example occur of microbial, viral, antibiotic or heavy metal origin. The contaminating substances could subsequently give extensive problems when the D-galactose preparation is used in for example food or feed applications.

Additionally, the D-galactose produced from milk products, cannot serve as an ingredient for foodstuffs prepared for people suffering from milk intolerance or foodstuffs prepared as kosher food. Especially the kosher food market is at present time a vast market.

The potential presence of contaminating substances, limited availability of milk and high costs related to milk production are the main causes of the high costs currently related to the production of D-galactose from milk. The current invention overcomes the aforementioned problems. A widely available alternative raw material is used in the process for the preparation of D-galactose according to the invention and in addition no extensive processing is needed for the removal of the indicated contaminating substances. Therefor the costs for manufacturing D-galactose and D-galactose comprising compositions are significantly reduced and D-galactose preparations can be utilized in a broader field of applications.

Besides the abovementioned commercially employed process using lactose as source for D-galactose production a number of alternative sources were suggested a long time ago. These processes were however not commercially viable as large scale processes. The skilled person has been concentrating on development of processes using lactose as source for the past two decades.

Vegetable raw materials described to date for the manufacturing of D-galactose have been selected on the presence of homologous galactose polymers, e.g. galactan. The rationale presumably being that only the hydrolysis of such homologous D-galactose polymers leads to useful D-galactose containing preparations. Because these polymers are usually present in the raw material in very low levels, economically feasible industrial scale processes for the production of D-galactose have however not been developed. In addition, no process using widely available vegetable raw materials has been developed.

Three examples of a process for the manufacture of D-galactose compositions using raw materials comprising homologous polymers of galactose is summarized below. The limited availability of raw material used in the processes, excessive use of chemicals and exotic climate required for the growth of the raw material ensure the processes are not economically feasible.

US 1718837 (1931) discloses a process for the preparation of D-galactose from the water extract of western larch wood (Larix occidentalis).

Banerji, in Journal of the Institution of Chemists (India) 52: 57-58 (1980) discloses a process for the preparation of D-galactose from green Aegle marmelos fruit-gum. The pretreatments for the preparation of D-galactose according to this process comprise: (i) isolation of the gum from bael-fruit by horizontally cutting the fruits and isolating the gum from the cavity along with the seeds with a small spatula (ii) dissolving the gum in water (iii) removal of the seeds by filtration (iv) addition of sulfuric acid to the filtrate to 0.1M (v) heating the acid solution at 100°C for 7 hours (vi) cooling the solution to room temperature, (vii) filtration of the solution (viii) pouring the filtrate in 5 volumes of acetone (ix) precipitating the D-galactan. Subsequently, pure D-galactan is mixed with sulfuric acid (4% w/w) and incubated overnight, after which the galactan is dissolved by heating the mixture to 80° to 85°C for 12 hours. Then water is added to the incubated mixture and said mixture is neutralized with barium hydroxide. After neutralization, the mixture is subjected to filtration and filtrate is passed through Amberlite IR-120 resins. The deionised solution is stirred with 1-2% active carbon, filtered and evaporated. Finally, the syrup is dissolved in methanol, water is added and the solution is placed in a refrigerator to allow crystallization of D-galactose.

The raw material source described above is not widely available and cannot be processed in the quantities required for current and future D-galactose needs. Additionally, the amount and identity of required processing aids needed in the process are quite harmful and environmentally hazardous. Furthermore, the process is very time consuming, labor intensive and costly.

Ingle, in Research and Industry, 21(4): 243-246 (1976) proposed a method for the preparation of D-galactose via hydrolysis of a concentrated aqueous extract of cashew nut shell with acid. The concentrated extract of cashew nut shells was acidified till the solution was 10% with respect to sulfuric acid. The acidic solution was refluxed for 12 hours followed by cooling and filtration. The residue was then washed with water and was rejected. The filtrate and the washing were combined and neutralized with barium hydroxide to a pH of 6.0. Further neutralization was done with barium carbonate to remove the free sulfuric acid present in the hydrolyzate. It could also be removed by passing the hydrolyzate through anion exchange resins, such as IRA-400. After washing the precipitate of barium sulfate with water, the neutral filtrate and washings were mixed and concentrated to syrup.

Obvious downsides of this process are the extensive amounts of chemicals needed (sulfuric acid, barium hydroxide, rectified spirit and activated charcoal) and the accompanying waste products produced. Additionally, the process uses considerable amounts of strong acids and alkali, which will damage processing equipment. These downsides make the process for the preparation of D-galactose from cashew nut shells unattractive and very costly.

Additionally, cashew nut shells are not available in the quantities heeded to fulfil the current and future industrial needs for D-galactose. The process according to the current invention uses a very limited amount of additional chemical substances, which greatly increase equipment lifetime, reduces manufacturing costs and reduces environmental pressure.

Considering the downsides of the known D-galactose production processes and the current and future needs for D-galactose, it remains highly desirable to develop a process for the manufacturing of D-galactose compositions from widely available vegetable sources, having high capacity, being easy to implement in processing industry, using limited amounts of processing aids, causing limited environmental pressure and still having limited manufacturing costs. Such a process is finally presented here.

As described earlier, when manufacturing D-galactose from vegetable sources on an industrial scale, to date, homologous polymers were proposed as a source of D-galactose. The process according to the invention however, does not use homologous galactose polymers as a source for D-galactose, but uses non-homologous sugar polymers as source for D-galactose. Surprisingly, such non-homologous sugars could be successfully hydrolyzed and D-galactose containing preparations could be obtained economically.

A provision to ensure a wide utilization of the D-galactose comprising preparation in for example foodstuffs, is that the preparation should comprise commonly accepted sugars as are often used in foodstuffs. The process according to invention thus uses non-homologous polysaccharide comprising at least a combination of D-galactose with D-glucose and /or D-fructose in at least 90% of the total of monosaccharide elements present in the oligosaccharides. Suitable oligosaccharides thus are melibiose, manninotriose, raffinose, stachyose and verbascose. Preferably the percentage of desired monosaccharide elements is as high as possible, preferably more than 95% even more preferably more than 99% of the monosaccharide elements present.

Rare sugars i.e. those sugars, which are not often used in foodstuffs and these are preferably absent from the oligosaccharides to be processed according to the invention. Rare sugars are for example arabinose, rhamnose, fucose, mannose, galactose variants other than D-galactose, type I arabinogalactan, type II arabinogalactan and uronic acids. These rare sugars are preferably absent because they are detrimental to application of D-galactose comprising compositions. The presence of such components would for example restrict the use of such preparations in food applications. Furthermore these contaminants could cause further problems in further purification of D-glucose.

Because both D-fructose and D-glucose are often used in the manufacturing of foodstuffs, preparations comprising D-galactose resulting from this process can be readily used in for example foodstuffs.

The preparations comprising D-galactose directly resulting from the process according to invention can suitably for example have (i) D-galactose and other components being mostly protein, D-glucose and/or D-fructose; (ii) D-galactose and other components being mostly D-fructose and/or D-glucose or (iii) an increased content of D-galactose, depending on the purity desired for the industrial utilization. Initially the process yields a D-galactose containing preparation that can be readily used in e.g. the food industry or fermentation industry. Additionally, this preparation can be subjected to additional purification steps, generating a composition with an elevated content of D-galactose, applicable in for example the food, cosmetic, fermentation or chemical industry.

It is well known that within legume seed, oligosaccharides, e.g. melibiose, manninotriose, raffinose, stachyose and verbascose, are present. These oligosaccharides are mainly regarded as waste because of their antinutritional properties in animal feed. However recently a niche market was recognised for these substances due to their usefulness within health foods as bifido-stimulating substances. Sometimes, the bifido-stimulating substances are treated to decrease sucrose content and thereby generate a more suitable low-caloric bifido-stimulating sweetener. None of the abovementioned processes have however been directed at producing mainly monosaccharides.

Due to the flatulence causing effect and thus the negative effect on the nutritional value of protein products, these oligosaccharides have to date been removed from the protein products manufactured from legume raw material. Removal of these oligosaccharides has, for example been accomplished by washing the protein product or degrading the flatulence causing saccharides in the protein composition. Sometimes, carbohydrate-degrading agents are added to the purified protein for improvement of protein compositions. With the process according to the invention, the to date discarded oligosaccharides of these known processes can now be used as a source for D-galactose and D-galactose comprising compositions.

### SUMMARY OF INVENTION

It has now been found that a surprisingly easy industrially applicable process can be used for the preparation of D-galactose from widely processed legume sources. In contrast to the previously described processes for the manufacturing of D-galactose from vegetable sources, the D-galactose preparations resulting from the process are not generated by the hydrolysis of homologous galactose polymers, but are generated by the hydrolysis of non-homologous sugar polymers, (commonly referred to as oligosaccharides) having at least 90% in total of monosaccharide elements present of D-galactose in combination with D-glucose and/or D-fructose. The composition of the oligosaccharides makes that preparations with varying content of D-galactose are very suitable for use in e.g. foodstuffs.

The D-galactose preparation can provide current and future industry with vast amounts of vegetable based D-galactose. Additionally, the costs for the preparation of D-galactose are significantly reduced compared to the currently known processes for the manufacturing of vegetable D-galactose and the process causes minimal environmental pressure when compared to processes currently known for the manufacturing of D-galactose from vegetable sources.

The process according to invention comprises (a) subjecting an oligosaccharide containing composition, wherein the oligosaccharide is composed of at least 90% in total of monosaccharide elements present of D-galactose in combination with D-glucose and/or D-fructose, to one or more treatments, resulting in a preparation comprising at least 30% of said oligosaccharides on dry weight basis and (b) hydrolyzing the said oligosaccharides of the preparation obtained in (a) into mainly monosaccharides. The monosacharides are preferably present as at least 60% of the total monosaccharide content derivable in theory from the oligosaccharides, more prefereably in more than 70%. Naturally as high a yield as possible is preferred such as at least 80%.

### DETAILED DESCRIPTION OF EXEMPLARY EMBODIMENTS OF THE INVENTION

A process for manufacturing a D-galactose preparation from legume source is described hereinafter.

In principle, any legume plant or plant part having a significant content of oligosaccharides, wherein the oligosaccharide is composed of at least 90% in total of monosaccharide elements present of D-galactose in combination with D-glucose and/or D-fructose, may come into consideration for use as a source of D-galactose. Such plants include oilseed vegetables and plants producing beans or peas having significant levels of said oligosaccharides. Table 1 gives some examples of legume sources having significant levels of oligosaccharides composed of at least 90% in total of monosaccharide elements present of D-galactose in chemical combination with D-glucose and/or D-fructose and which are therefore good sources for preparation of D-galactose containing compositions. The percentages of said oligosaccharides, are greatly dependent on for example growth conditions and plant species used.

In Table 1, the percentage of said oligosaccharides, is calculated as dry weight of said oligosaccharides per total dry weight of the D-galactose source. The percentage D-galactose is calculated as dry weight of D-galactose after total hydrolysis of said oligosaccharides per total dry weight before hydrolysis of the oligosaccharides.

**TABLE 1**

| D-galactose source | Oligosaccharides | Percentage D-galactose |
|---|---|---|
| Sunflower | 8-12% | 10-30% |
| Rape | 7-14% | 5-25% |
| Lupin | 6-14% | 20-60% |
| Soybean | 5-10% | 20-40% |
| Cowpeas | 5-15% | 30-60% |

The source used as the starting material in the present process may, of course, be derived from two or more plants and/or plant parts. Furthermore, the source of said oligosaccharides may have been derived from the original plant or plant part via appropriate preliminary treatments such as dehulling or removal of husk or such like, flaking, the removal of at least part of the fat or oil content, and/or milling, grinding or commution. Furthermore, preliminary treatment might include the removal of at least part of the protein, fibers or starch present. The said material may be in any suitable form, e.g. grits, flakes, flour or meal.

At present, particularly preferred starting materials that contain said oligosaccharides are rapeseed, sunflower seed and soybean, especially flakes, flour or meal thereof, for example defatted flakes, flour or meal.

In certain preferred embodiments, the oligosaccharides are extracted from a vegetable source using aqueous extractant, with or without water-soluble organic solvents and/or with or without dissolved salts. The extract comprising said oligosaccharides may contain a high content of protein, fiber or starch when compared to oligosaccharides. When said extract has high protein, fiber or starch contents, additional treatment before hydrolysis of said oligosaccharides, in the form of removal of protein, fiber or starch can be desired. Treatment for the removal of these components can be any suitable treatment known in the art. The treatment can for example comprise extraction, centrifugation, decanting or membrane filtration. Such treatments can be carried out singly or in combination. They can also be further combined with other techniques like isoelectric protein precipitation. Such treatments are also well known to the skilled person.

In certain preferred embodiments defatted legume material is mixed with water to solvatize the desired oligosaccharides. From this mixture, the insoluble phase is removed from the soluble phase e.g. by decanting. The soluble phase is then treated to precipitate the proteins, while the oligosaccharides remain solvated. This can for example occur using acid. The insolubilized proteins may then be removed e.g. by centrifugation. The preparation thus obtained comprises at least 30% of the oligosaccharides on dry weight basis. The oligosaccharides in the soluble phase are then subsequently hydrolyzed. The hydrolysis can occur in a manner known per se for oligosaccharide hydrolysis e.g. by acid treatment or by applying enzymatic action for releasing monosaccharides from the specific oligosaccharides present in the preparation. Enzymatic treatment forms a preferred embodiment in the process according to the invention.

In certain preferred embodiments, enzymes having the ability to break both alpha-galactosidic linkage and the ability to break beta-fructofuranosidic linkage or a mixture of enzymes comprising the ability to break alpha-galactosidic linkage and the ability to break beta-fructofuranosidic linkage are added to the extracted fraction as a hydrolyzing agent.

In a process accordingto the invention at least 30% of the oligosaccharides on dry weight basis is required for hydrolysis to be efficient. When using enzyme preparations as a hydrolyzing agent the quantity of enzyme used will have great impact on the manufacturing costs of D-galactose containing compositions. When applying enzymes as a hydrolyzing agent to solutions having a content of oligosaccharides lower than 30% on dry weight basis, either a prohibitive additional amount of enzyme would have to be added or the required incubation time would have to be increased by an unacceptable amount. This would have a negative impact on the manufacturing costs. Another potential downside of the application of hydrolyzing action in preparations comprising less than 30% of said oligosaccharides on dry weight basis is that the hydrolyzing agents are given the opportunity to act on other components than said oligosaccharides. This could cause the final preparation to be contaminated with rare sugars, for example arabinose, rhamnose, fucose, mannose, or uronic-acids. These rare sugars are unwanted because they damage the applicability of the D-galactose comprising compositions. The presence of these components will limit the scope of for example food applications in which the compositions can be utilized. Furthermore, these contaminants cause problems in further purification of D-galactose.

In certain preferred embodiments, the hydrolysis is accomplished by holding the mixture comprising enzymatic hydrolyzing agent and said oligosaccharides at a holding temperature between 10°C and 90°C for 5 to 250 minutes, more preferred at a holding temperature between 20°C and 60°C for 10 to 100 minutes.

In certain preferred embodiments, the said oligosaccharides present in the soluble fraction after removal of precipitated protein are further purified before a hydrolyzing agent is added. Further purification of said oligosaccharides before hydrolysis may be accomplished using any convenient separation technique, for example membrane separation techniques such as ultrafiltration, diafiltration, microfiltration, nanofiltration, hyperfiltration, etc. or chromatographic techniques or a combination thereof. When using ultrafiltration, the membranes used can have a theoretical molecular weight cut-off of about 1,000 to about 200,000 Dalton, more preferred from about 2,000 to about 50,000 Dalton, most preferred 5,000 to 35,000 Dalton.

When the application, for which the D-galactose compositions is required, needs a relatively pure D-galactose preparation, the concentration of D-galactose can be elevated using separation techniques, for example membrane separation techniques, chromatographic techniques or combinations thereof.

Increase of D-galactose content can be accomplished by separating D-galactose from other saccharides or by separating D-galactose from non-saccharides or combinations thereof.

In certain preferred embodiments, first the (residual) non-saccharides are separated from the D-galactose. The resulting saccharide mix comprises mainly monosaccharides and can be used in various types of industry. Subsequently, the content of D-galactose can be further increased by removal of the other saccharides. The rest streams of this process will thus be enriched in other monosaccharides such as D-fructose and D-glucose. These monosaccharides can also be sold as a valuable raw material in various industries. D-galactose can for example be separated from such other saccharides using techniques known in the art to the skilled person. Using techniques known in the art of separating glucose/galactose mixtures obtained via the hydrolysis of lactose on industrial or at least comercially attractive scale provides a suitable embodiment of the process according to the invention which provides good results.

In a suitable embodiment, the D-galactose and other saccharides are separated .using column chromatography. Additionally, chromatography can also be used for increasing the concentration of D-galactose in the preparation by removal of salt, protein or fibrous components using known technology.

Also techniques using active charcoal and crystallization can be used to increase the D-galactose content.

The process according to the invention is to be carried out at a commercially attractive scale i.e. at least on a scale equal to pilot scale. In the examples pilot scale tests are illustrated.

### EXAMPLES

The following examples are not intended to limit or narrow the scope of the invention described herein, but are for illustrative purposes only.

### Example 1:

On a pilot plant scale, 150-liters of water having a temperature of about 20°C was added to 30 kg defatted soybean flakes and stirred to form a uniform mixture. After 20 minutes, insoluble components in said mixture were separated from the soluble fraction by decanting. Hydrochloric acid was added to the soluble fraction to conduct acid precipitation at pH 4.5, followed by centrifugation at 7800xg to separate the insolubles from the solubles. The soluble fraction was further treated using ultrafiltration. The ultrafiltration membrane had a theoretical molecular weight cut-off of 5.000 Dalton. The retentate was separated from the permeate, which contained soluble saccharides. The permeate contained about 50% saccharides on dry weight basis.
0.1% Alpha-gal 600L (Novo-Nordisk) based on dry matter was added. (Alpha-gal 600L is an enzyme preparation containing both alpha-galactosidic activity and beta-fructofuranosidic activity). The polysaccharides within the permeate soluble saccharides on dry weight basis were hydrolyzed to a preparation containing mainly monosaccharides, by incubating the mixture for 4 hours at 50°C. The D-galactose content of the preparation thus obtained was about 5-10% on dry weight basis.

### Example 2:

On a pilot plant scale, 150 liters of water having a temperature of about 20°C was added to 30 kg defatted soybean flakes and stirred to form a uniform mixture. After 20 minutes insoluble components in said mixture were separated from the soluble fraction by decanting. Hydrochloric acid was added to the soluble fraction to adjust pH to about 4.5. The formed precipitate was removed by centrifugation at 7800xg. The remaining soluble fraction thus obtained contained at least 30% oligosaccharides on a dry weight.
0.1% Alpha-gal 600L based on dry matter was added. The polysaccharides within the permeate soluble saccharides on dry weight basis were hydrolyzed to a preparation containing mainly monosaccharides, by incubating the mixture for 4 hours at 50°C. The D-galactose content of the preparation thus obtained was about 5% on a dry weight basis.

### Example 3:

According to the same manner as described in Example 2, the preparation containing about 5% D-galactose on dry weight basis was obtained.
The preparation was then further treated using ultrafiltration. The ultrafiltration membrane had a theoretical molecular weight cut-off of 5.000 Dalton. The retentate was separated from the permeate, which contained soluble saccharides. The permeate contained about 5-10% D-galactose on dry weight basis.

### Example 4:

On a pilot plant scale, 150 liters of water having a temperature of about 20°C was added to 30 kg defatted soybean flakes and stirred to form a uniform mixture. After 20 minutes, insoluble components in said mixture were separated from the soluble fraction by decanting. Hydrochloric acid was added to the soluble fraction to conduct acid precipitation at pH 4.5, followed by centrifugation at 7800xg to separate the insolubles from the solubles. The remaining soluble fraction contained about 50% saccharides on dry weight basis.

1000 ml of the soluble fraction containing about 50% saccharides on dry weight basis was applied to a chromatography column, filled with 100 ml Purolite Macronet MN 500 resin. at a flow rate of 3 bed volumes per hour and a temperature 60°C. Fractions were collected until the column was unable to bind anymore protein or color causing components. Protein content, ash content and color of the collected fractions were significantly reduced. The 500 ml pooled fraction after this chromatographic step contained about 75% soluble saccharides on dry weight basis.

0.1% Alpha-gal 600L based on dry matter was added. The polysaccharides within the permeate soluble saccharides on dry weight basis were hydrolyzed to a preparation containing mainly monosaccharides, by incubating the mixture for 4 hours at 50°C. The D-galactose content of the preparation thus obtained was increased by at least 50% on a dry weight basis in comparison to the yield of methods according to examples 1, 2 or 3.

### Example 5:

On laboratory scale, 1-liter water of about 20°C was added to 200 gram defatted sunflower meal and stirred to form a uniform mixture. After 20 minutes, the mixture was filtered over a screen having a pore size of 100µm. To the permeate hydrochloric acid was added to conduct acid precipitation at about pH 4.2, followed by centrifugation at 4696xg to separate the insolubles from the solubles. The remaining soluble fraction contained at least 30% saccharides on dry weight basis.

0.1% Alpha-gal 600L based on dry matter was added. The polysaccharides within the permeate soluble saccharides on dry weight basis were hydrolyzed to a preparation containing mainly monosaccharides, by incubating the mixture for 4 hours at 50°C. The D-galactose content of the preparation thus obtained was about 3% on a dry weight basis. These initial results indicate that the use of sunflower as source should provide similar results to the method using soybean. The results can be optimised as indicated in the other examples and description to further increase the yield and/or purity.

### Example 6:

On laboratory scale, 1.5-liter water of about 20°C was added to 150 gram defatted rapeseed meal and stirred to form a uniform mixture. After 20 minutes, the mixture was filtered over a screen having a pore size of 100µm. To the permeate hydrochloric acid was added to conduct acid precipitation at about pH 4.5. followed by centrifugation at 4696xg to separate the insolubles from the solubles. The remaining soluble fraction contained about 35% saccharides on dry weight basis.

0.1% Alpha-gal 600L based on dry matter was added. The polysaccharides within the permeate soluble saccharides on dry weight basis were hydrolyzed to a preparation containing mainly monosaccharides, by incubating the mixture for 4 hours at 50°C. The D-galactose content of the preparation thus obtained was about 3.5% on a dry weight basis. These initial results indicate that the use of rapeseed as source should provide similar results to the method using soybean. The results can be optimised as indicated in the other examples and description to further increase the yield and/or purity.

### FIGURE DESCRIPTION

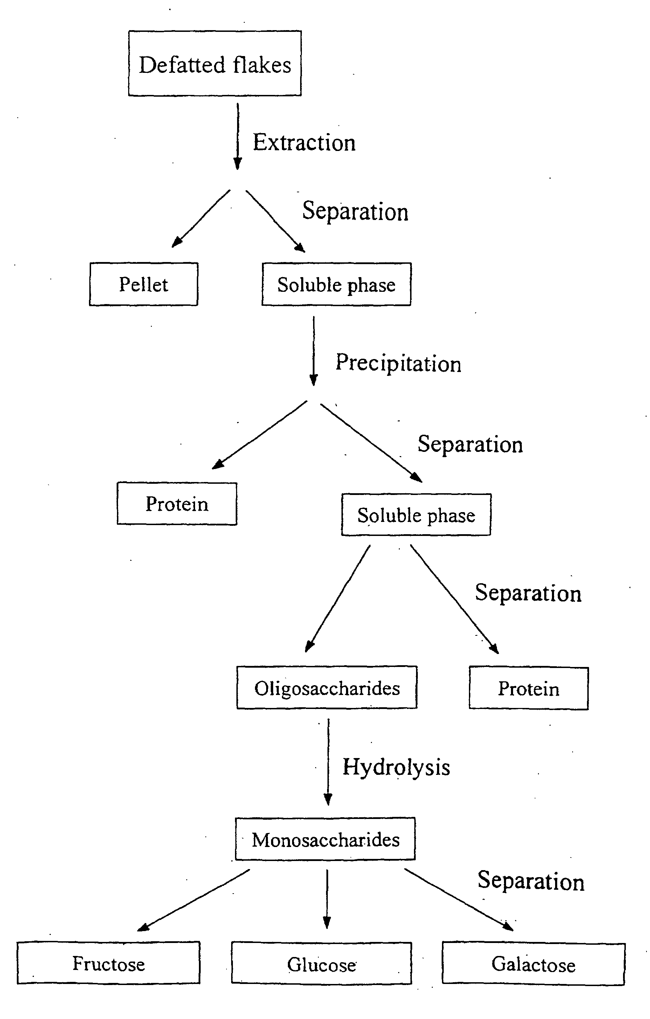

Figure 1shows a flow sheet of an embodiment of the process according to the invention.

## Claims

1. A process for manufacturing D-galactose from an oligosaccharide containing legume composition, comprising the steps of:
a) subjecting an oligosaccharide containing legume composition, wherein at least 90% of the total monosaccharide elements of the oligosaccharide are D-galactose in chemical combination with D-glucose and/or D-fructose, to one or more treatments, resulting in a preparation comprising at least 30% of said oligosaccharides on dry weight basis
b) hydrolyzing the said oligosaccharides of the preparation obtained in a) into mainly monosaccharides.

2. A process according to claim 1, wherein at least 60% of the oligosaccharides of the preparation obtained in a) are hydrolyzed into monosaccharides.

3. A process according to claim 1, wherein at least 70% of the oligosaccharides of the preparation obtained in a) are hydrolyzed into monosaccharides.

4. A process according to claim 1, wherein at least 80% of the oligosaccharides of the preparation obtained in a) are hydrolyzed into monosaccharides.

5. A process according to any of claims 1-4, wherein the treatment of the oligosaccharide containing composition according to step a) results in a preparation comprising at least 40% oligosaccharides on dry weight basis.

6. A process according to any of claims 1-5, wherein the treatment of oligosaccharide containing composition according to step a) results in a preparation comprising at least 80% oligosaccharides on dry weight basis.

7. A process according to any of claims 1-6, wherein the legume plant source for oligosaccharides is soy, rape or sunflower or a mixture thereof.

8. A process according to any of claims 1-7, wherein the oligosaccharides are extracted from defatted beans, peas or oilseeds, preferably from defatted soybean preparations, defatted rapeseed preparations, defatted sunflower seed preparations or mixtures thereof.

9. A process according to any of claims 1-8, wherein the oligosaccharides consist of at least 90% of oligosaccharides selected from any of the following group as such or in combination, said group consisting of melibiose, manninotriose, raffinose, stachyose, verbascose.

10. A process according to any of claims 1-9, wherein the oligosaccharides are extracted using an aqueous solution, with or without water-soluble organic solvents and/or with or without dissolved salts and/or with or without acids.

11. A process according to claim 10, wherein the aqueous solution is water.

12. A process according to any of claims 1-11, wherein the treatment of oligosaccharide containing composition according to step a) comprises the removal of non-saccharides.

13. A process according to any of claims 1-12, wherein the treatment of oligosaccharide containing composition according to step a) comprises extraction followed by separation of the resulting soluble and insoluble phases and subjecting the soluble phase to further treatment.

14. A process according to any of claims 1-13, wherein the treatment of oligosaccharide containing composition according to step a) comprises acid precipitation followed by separation of the resulting soluble and insoluble phases and subjecting the soluble phase to further treatment.

15. A process according to any of claims 1-14, wherein the treatment of oligosaccharide containing composition according to step a) comprises use of membrane separation techniques for removing non saccharides.

16. A process according to claim 15, wherein the membrane separation technique used is ultrafiltration.

17. A process according to claim 16, wherein the ultrafiltration membrane used has a theoretical molecular weight cut-off of about 1,000 to about 200,000 Dalton, preferably 5,000-20,000.

18. A process according to any of claims 1-17, wherein the hydrolysis of oligosaccharides is accomplished using enzymatic hydrolysis.

19. A process according to claim 18 wherein the enzymatic hydrolysis is accomplished by applying an enzyme having the ability to break 1-6-α-galactosidic linkage.

20. A process according to claim 19 wherein the enzymatic hydrolysis is accomplished by applying an enzyme having the ability to break 1-2-β-fructofuranosidic linkage.

21. A process according to claim 20 or 21, wherein the enzymatic hydrolysis is accomplished by applying a mixture of enzymes having the ability to break 1-6-α-galactosidic linkage and 1-2-β-fructofuranosidic linkage.

22. A process according to any of claims 1-21, comprising one or more further treatments following step b) for increasing the D-galactose concentration.

23. A process according to claim 22 wherein the further treatment comprises the removal of components other than D-galactose.

24. A process according to claim 22 or 23, wherein the further treatment comprises the removal of non-saccharides.

25. A process according to any of claims 22-24, wherein the further treatment is a chromatographic treatment.

26. A process according to any of claims 22-25, wherein the further treatment comprises the removal of other saccharides.

27. A process according to any of claims 22-26, wherein the further treatment comprises the removal of D-glucose and/or D-fructose.

28. A process according to any of claims 1-27, wherein the hydrolysis of oligosaccharides occurs at a holding temperature of 10° to 90°C for 5 to 250 minutes.

29. A process according to any of claims 1-28, wherein the hydrolysis of oligosaccharides occurs at a holding temperature of 25° to 50°C for 10 to 100 minutes.

30. A process according to any of claims 1-29, resulting in a composition comprising at least 5 % D-galactose on a dry weight basis.

31. A process according to any of claims 1-30, resulting in a composition comprises at least 10 % D-galactose on a dry weight basis.

32. A process according to any of claims 1-31, resulting in a composition comprises at least 40 % D-galactose on a dry weight basis.

33. A process according to any of claims 1-32, resulting in a composition comprises at least 90 % D-galactose on a dry weight basis.

34. A process according to any of the preceeding claims carried out at a scale at least equivalent to pilot scale, preferably equivalent to industrial scale.

## Patentansprüche

1. Verfahren zur Herstellung von D-Galactose aus einer oligosaccharidhaltigen Leguminosenzusammensetzung, umfassend die Schritte:
(a) Unterwerfen einer oligosaccharidhaltigen Leguminosenzusammensetzung, worin zumindest 90 % der Gesamtmonosaccharidelemente des Oligosaccharids D-Galactose in chemischer Kombination mit D-Glucose und/oder D-Fructose sind, einer oder mehreren Behandlungen, die zu einem Präparat, umfassend zumindest 30 % der Oligosaccharide auf Trockengewichtsbasis, führen,
(b) Hydrolysieren der Oligosaccharide des in (a) erhaltenen Präparats hauptsächlich zu Monosachariden.

2. Verfahren gemäss Anspruch 1, worin zumindest 60 % der Oligosaccharide des in (a) erhaltenen Präparats zu Monosacchariden hydrolysiert werden.

3. Verfahren gemäss Anspruch 1, worin zumindest 70 % der Oligosaccharide des in (a) erhaltenen Präparats zu Monosacchariden hydrolysiert werden.

4. Verfahren gemäss Anspruch 1, worin zumindest 80 % der Oligosaccharide des in (a) erhaltenen Präparats zu Monosacchariden hydrolysiert werden.

5. Verfahren gemäss einem der Ansprüche 1 bis 4, worin die Behandlung der oligosaccharidhaltigen Zusammensetzung gemäss Schritt (a) zu einem Präparat, umfassend zumindest 40 % Oligosaccharide auf Trockengewichtsbasis, führt.

6. Verfahren gemäss einem der Ansprüche 1 bis 5, worin die Behandlung der oligosaccharidhaltigen Zusammensetzung gemäss Schritt (a) zu einem Präparat, umfassend zumindest 80 % Oligosaccharide auf Trockengewichtsbasis, führt.

7. Verfahren gemäss einem der Ansprüche 1 bis 6, worin die Leguminosen-Pflanzenquelle für die Oligosaccharide Soja, Raps oder Sonnenblume oder eine Mischung davon ist.

8. Verfahren gemäss einem der Ansprüche 1 bis 7, worin die Oligosaccharide aus entfetteten Bohnen, Erbsen oder Ölsamen, bevorzugt aus entfetteten Sojabohnenpräparaten, entfetteten Rapssamenpräparaten, entfetteten Sonnenblumensamenpräparaten oder Mischungen davon extrahiert werden.

9. Verfahren gemäss einem der Ansprüche 1 bis 8, worin die Oligosaccharide zumindest zu 90 % aus Oligosacchariden bestehen, die aus jedem aus der folgenden Gruppe, einzeln oder in Kombination, ausgewählt sind, wobei die Gruppe aus Melibiose, Manninotriose, Raffinose, Stachyose oder Verbascose besteht.

10. Verfahren gemäss einem der Ansprüche 1 bis 9, worin die Oligosaccharide unter Verwendung einer wässrigen Lösung, mit oder ohne wasserlösliche organische Lösungsmittel und/oder mit oder ohne gelöste Salze und/oder mit oder ohne Säuren extrahiert werden.

11. Verfahren gemäss Anspruch 10, worin die wässrige Lösung Wasser ist.

12. Verfahren gemäss einem der Ansprüche 1 bis 11, worin die Behandlung der oligosaccharidhaltigen Zusammensetzung gemäss Schritt (a) die Entfernung der Nicht-Saccharide umfasst.

13. Verfahren gemäss einem der Ansprüche 10 bis 12, worin die Behandlung der oligosaccharidhaltigen Zusammensetzung gemäss Schritt (a) Extraktion, gefolgt von Trennung der resultierenden löslichen und unlöslichen Phasen und Unterwerfen der löslichen Phase der weiteren Behandlung umfasst.

14. Verfahren gemäss einem der Ansprüche 1 bis 13, worin die Behandlung der oligosaccharidhaltigen Zusammensetzung gemäss Schritt (a) Säurepräzipitation, gefolgt von Trennung der resultierenden löslichen und unlöslichen Phasen und Unterwerfen der löslichen Phase der weiteren Behandlung umfasst.

15. Verfahren gemäss einem der Ansprüche 1 bis 14, worin die Behandlung der oligosaccharidhaltigen Zusammensetzung gemäss Schritt (a) die Verwendung von Membrantrennungstechniken zum Entfernen der Nicht-Saccharide umfasst.

16. Verfahren gemäss Anspruch 15, worin die verwendete Membrantrennungstechnik Ultrafiltration ist.

17. Verfahren gemäss Anspruch 16, worin die verwendete Ultrafiltrationsmembran einen theoretischen Molekulargewichts-Cut-Off von etwa 1.000 bis etwa 200.000 Dalton, bevorzugt 5.000 bis 20.000, hat.

18. Verfahren gemäss einem der Ansprüche 1 bis 17, worin die Hydrolyse der Oligosaccharide unter Verwendung von enzymatischer Hydrolyse bewerkstelligt wird.

19. Verfahren gemäss Anspruch 18, worin die enzymatische Hydrolyse durch Anwendung eines Enzyms mit der Fähigkeit, 1-6-α-galactosidische Bindungen zu brechen, bewerkstelligt wird.

20. Verfahren gemäss Anspruch 19, worin die enzymatische Hydrolyse durch Anwendung eines Enzyms mit der Fähigkeit, 1-2-β-fructofuranosidische Bindungen zu brechen, bewerkstelligt wird.

21. Verfahren gemäss Anspruch 20 oder 21, worin die enzymatische Hydrolyse durch Anwendung einer Mischung von Enzymen mit der Fähigkeit, 1-6-α-galactosidische Bindungen und 1-2-β-fructofuranosidische Bindungen zu brechen, bewerkstelligt wird.

22. Verfahren gemäss einem der Ansprüche 1 bis 21, umfassend eine oder mehrere Behandlungen nach Schritt (b) zur Erhöhung der D-Galactosekonzentration.

23. Verfahren gemäss Anspruch 22, worin die weitere Behandlung die Entfernung von anderen Bestandteilen als D-Galactose umfasst.

24. Verfahren gemäss Anspruch 22 oder 23, worin die weitere Behandlung die Entfernung von Nicht-Sacchariden umfasst.

25. Verfahren gemäss einem der Ansprüche 22 bis 24, worin die weitere Behandlung eine chromatografische Behandlung ist.

26. Verfahren gemäss einem der Ansprüche 22 bis 25, worin die weitere Behandlung die Entfernung von anderen Sacchariden umfasst.

27. Verfahren gemäss einem der Ansprüche 22 bis 26, worin die weitere Behandlung die Entfernung von D-Glucose und/oder D-Fructose umfasst.

28. Verfahren gemäss einem der Ansprüche 1 bis 27, worin die Hydrolyse der Oligosaccharide bei einer Haltetemperatur von 10 bis 90°C für 5 bis 250 Minuten stattfindet.

29. Verfahren gemäss einem der Ansprüche 1 bis 28, worin die Hydrolyse der Oligosaccharide bei einer Haltetemperatur von 25 bis 50°C für 10 bis 100 Minuten stattfindet.

30. Verfahren gemäss einem der Ansprüche 1 bis 29, das zu einer Zusammensetzung, umfassend zumindest 5 % D-Galactose auf Trockengewichtsbasis, führt.

31. Verfahren gemäss einem der Ansprüche 1 bis 30, das zu einer Zusammensetzung, umfassend zumindest 10 % D-Galactose auf Trockengewichtsbasis, führt.

32. Verfahren gemäss einem der Ansprüche 1 bis 31, das zu einer Zusammensetzung, umfassend 40 % D-Galactose auf Trockengewichtsbasis, führt.

33. Verfahren gemäss einem der Ansprüche 1 bis 32, das zu einer Zusammensetzung, umfassend 90 % D-Galactose auf Trockengewichtsbasis, führt.

34. Verfahren gemäss einem der vorangehenden Ansprüche, das in einem Massstab durchgeführt wird, der zumindest dem Pilotmassstab, bevorzugt dem industriellen Massstab, äquivalent ist.

## Revendications

1. Procédé pour la production de D-galactose à partir d'une composition de légume contenant des oligosaccharides, comprenant les étapes consistant :
a) à soumettre une composition de légume contenant des oligosaccharides, dans laquelle au moins 90% des éléments monosaccharidiques totaux des oligosaccharides consistent en D-galactose en association chimique avec le D-glucose et/ou le D-fructose, à un ou plusieurs traitements, avec pour résultat une préparation comprenant au moins 30% desdits oligosaccharides sur base pondérale sèche,
b) à hydrolyser lesdits oligosaccharides dans la préparation obtenue en a) en monosaccharides principalement.

2. Procédé suivant la revendication 1, dans lequel au moins 60% des oligosaccharides dans la préparation obtenue en a) sont hydrolysés en monosaccharides.

3. Procédé suivant la revendication 1, dans lequel au moins 70% des oligosaccharides de la préparation obtenue en a) sont hydrolysés en monosaccharides.

4. Procédé suivant la revendication 1, dans lequel au moins 80% des oligosaccharides de la préparation obtenue en a) sont hydrolysés en monosaccharides.

5. Procédé suivant l'une quelconque des revendications 1 à 4 dans lequel le traitement de la composition contenant des oligosaccharides suivant l'étape a) a pour résultat une préparation comprenant au moins 40% d'oligosaccharides sur base pondérale sèche.

6. Procédé suivant l'une quelconque des revendications 1 à 5, dans lequel le traitement de la composition contenant des oligosaccharides suivant l'étape a) a pour résultat une préparation comprenant au moins 80% d'oligosaccharides sur base pondérale sèche.

7. Procédé suivant l'une quelconque des revendications 1 à 6, dans lequel la source végétale consistant en légumes pour les oligosaccharides est le soja, le colza, le tournesol ou un de leurs mélanges.

8. Procédé suivant l'une quelconque des revendications 1 à 7, dans lequel les oligosaccharides sont extraits de haricots, de pois ou de graines oléagineuses dégraissés de préférence, de préparations de soja dégraissées, de préparations de colza dégraissées, de préparations de graines de tournesol dégraissées ou de leurs mélanges.

9. Procédé suivant l'une quelconque des revendications 1 à 8, dans lequel les oligosaccharides consistent en au moins 90% d'oligosaccharides choisis dans n'importe lequel des groupes suivants tels quels ou en association, ledit groupe consistant en mélibiose, manninotriose, raffinose, stachyose, verbascose.

10. Procédé suivant l'une quelconque des revendications 1 à 9, dans lequel les oligosaccharides sont extraits en utilisant une solution aqueuse avec ou sans solvants organiques hydrosolubles et/ou avec ou sans sels dissous et/ou avec ou sans acides.

11. Procédé suivant la revendication 10, dans lequel la solution aqueuse est l'eau.

12. Procédé suivant l'une quelconque des revendications 1 à 11, dans lequel le traitement de la composition contenant des oligosaccharides suivant l'étape a) comprend l'élimination des non-saccharides.

13. Procédé suivant l'une quelconque des revendications 1 à 12, dans lequel le traitement de la composition contenant des oligosaccharides suivant l'étape a) comprend une extraction suivie par une séparation de la phase soluble et de la phase insoluble résultantes et l'étape consistant à soumettre à la phase soluble un traitement supplémentaire.

14. Procédé suivant l'une quelconque des revendications 1 à 13, dans lequel le traitement de la composition contenant des oligosaccharides suivant l'étape a) comprend une précipitation par un acide suivie par une séparation de la phase soluble et de la phase insoluble résultantes et l'étape consistant à soumettre la phase soluble à un traitement supplémentaire.

15. Procédé suivant l'une quelconque des revendications 1 à 14, dans lequel le traitement de la composition contenant des oligosaccharides suivant l'étape a) comprend l'utilisation des techniques de séparation par membrane pour éliminer les non-saccharides.

16. Procédé suivant la revendication 15, dans lequel la technique de séparation par membrane utilisée est l'ultrafiltration.

17. Procédé suivant la revendication 16, dans lequel la membrane d'ultrafiltration utilisée a une valeur d'exclusion de poids moléculaire théorique d'environ 1000 à environ 200 000 daltons, de préférence dé 5000 à 20 000.

18. Procédé suivant l'une quelconque des revendications 1 à 17, dans lequel l'hydrolyse des oligosaccharides est effectuée par hydrolyse enzymatique.

19. Procédé suivant la revendication 18, dans lequel l'hydrolyse enzymatique est effectuée en appliquant une enzyme ayant aptitude à rompre la liaison 1-6-α-galactosidique.

20. Procédé suivant la revendication 19, dans lequel l'hydrolyse enzymatique est effectuée en appliquant une enzyme ayant l'aptitude à rompre la liaison 1-2-β-fructofuranosidique.

21. Procédé suivant la revendication 20 ou 21, dans lequel l'hydrolyse enzymatique est effectuée en appliquant un mélange d'enzymes ayant l'aptitude à rompre la liaison 1-6-α-galactosidique et la liaison 1-2-β-fructofuranosidique.

22. Procédé suivant l'une quelconque des revendications 1 à 21, comprenant un ou plusieurs traitements supplémentaires après l'étape b) pour augmenter la concentration en D-galactose.

23. Procédé suivant la revendication 22, dans laquelle le traitement supplémentaire comprend l'élimination des constituants autres que le D-galactose.

24. Procédé suivant la revendication 22 ou 23, dans lequel le traitement supplémentaire comprend l'élimination des non-saccharides.

25. Procédé suivant l'une quelconque des revendications 22 à 24, dans lequel le traitement supplémentaire est un traitement chromatographique.

26. Procédé suivant l'une quelconque des revendications 22 à 25, dans lequel le traitement supplémentaire comprend l'élimination d'autres saccharides.

27. Procédé suivant l'une quelconque des revendications 22 à 26, dans lequel le traitement supplémentaire comprend l'élimination du D-glucose et/ou du D-fructose.

28. Procédé suivant l'une quelconque des revendications 1 à 27, dans lequel l'hydrolyse des oligosaccharides s'effectue à une température de maintien de 10° à 90°C pendant un temps de 5 à 250 minutes.

29. Procédé suivant l'une quelconque des revendications 1 à 28, dans lequel l'hydrolyse des oligosaccharides se produit à une température de maintien de 25° à 50°C pendant 10 à 100 minutes.

30. Procédé suivant l'une quelconque des revendications 1 à 29, ayant pour résultat une composition comprenant au moins 5% de D-galactose sur base pondérale sèche.

31. Procédé suivant l'une quelconque des revendications 1 à 30, ayant pour résultat une composition comprenant au moins 10% de D-galactose sur base pondérale sèche.

32. Procédé suivant l'une quelconque des revendications 1 à 31, ayant pour résultat une composition comprenant au moins 40% de D-galactose sur base pondérale sèche.

33. Procédé suivant l'une quelconque des revendications 1 à 32, ayant pour résultat une composition comprenant au moins 90%. de D-galactose sur base pondérale sèche.

34. Procédé suivant l'une quelconque des revendications précédentes, mis en oeuvre à une échelle au moins équivalente à une échelle pilote, de préférence équivalente à l'échelle industrielle.
